# FASCICULE DE BREVET EUROPEEN

(11) **EP 2 117 566 B1**
(45) Date de publication et mention de la délivrance du brevet: **19.10.2016**
(21) Numéro de dépôt: 08761845.0
(22) Date de dépôt: 06.02.2008
(51) Int. Cl.: A61K 35/02, A61P 1/12, A61P 37/02

(54) **UTILISATION D'ARGILES POUR LE TRAITEMENT DE LA MALADIE COELIAQUE**
VERWENDUNG VON TONMINERALIEN ZUR BEHANDLUNG DER ZÖLIAKIE
USE OF CLAY FOR TREATING COELIAC DISEASE

(30) Priorité: 06.02.2007 FR 0700821
(43) Date de publication de la demande: 18.11.2009
(73) Titulaire: IPSEN PHARMA S.A.S., 91200 Boulogne-Billancourt (FR)
(72) Inventeur: MATHIEX-FORTUNET, Hélène, F-75116 Paris (FR)
(74) Mandataire: Audonnet, Nathalie
(86) Numéro de dépôt international: PCT/FR2008/000142
(87) Numéro de publication internationale: WO 2008/113905

(56) Documents cités:
- FR-A1- 2 581 874
- YEN ZUI-SHEN ET AL: "Best evidence topic report. Smectite for acute diarrhoea in children." EMERGENCY MEDICINE JOURNAL : EMJ JAN 2006, vol. 23, no. 1, janvier 2006 (2006-01), pages 65-66, XP008084193 ISSN: 1472-0213
- YAO-ZONG Y ET AL: "Comparative efficacy of dioctahedral smectite (Smecta<(>R)) and a probiotic preparation in chronic functional diarrhoea" DIGESTIVE AND LIVER DISEASE, W.B. SAUNDERS, vol. 36, no. 12, décembre 2004 (2004-12), pages 824-828, XP002452936 ISSN: 1590-8658
- GONZÁLEZ RAQUEL ET AL: "Anti-inflammatory effect of diosmectite in hapten-induced colitis in the rat." BRITISH JOURNAL OF PHARMACOLOGY MAR 2004, vol. 141, no. 6, mars 2004 (2004-03), pages 951-960, XP002452651 ISSN: 0007-1188
- DUCROTTE P ET AL: "Symptomatic efficacy of beidellitic montmorillonite in irritable bowel syndrome: A randomized, controlled trial" ALIMENTARY PHARMACOLOGY AND THERAPEUTICS 15 FEB 2005 UNITED KINGDOM, vol. 21, no. 4, 15 février 2005 (2005-02-15), pages 435-444, XP002452653 ISSN: 0269-2813
- DATABASE WPI Week 200602 Thomson Scientific, London, GB; AN 2006-019993 XP002453043 & WO 2005/112881 A1 (SHANDONG LUYE PHARM CO LTD) 1 décembre 2005 (2005-12-01)
- THEODOROU V ET AL: "Protective action of diosmectite treatment on digestive disturbances induced by intestinal anaphylaxis in the guinea-pig" ALIMENTARY PHARMACOLOGY AND THERAPEUTICS 1994 UNITED KINGDOM, vol. 8, no. 3, 1994, pages 295-299, XP008084198 ISSN: 0269-2813
- LEONARD A ET AL: "Pepsin hydrolysis of the adherent mucus barrier and subsequent gastric mucosal damage in the rat: Effect of diosmectite and 16,16 dimethyl prostaglandin E2" GASTROENTEROLOGIE CLINIQUE ET BIOLOGIQUE 1994 FRANCE, vol. 18, no. 6-7, 1994, pages 609-616, XP008084211 ISSN: 0399-8320
- CHANG F-Y ET AL.,: "Efficacy of dioctahedral smectite in treating patients of diarrhea-predominant irritable bowel syndrome" JOURNAL OF GASTROENTEROLOGY AND HEPATOLOGY, [Online] - 2007 pages 1-7, XP002452937 Extrait de l'Internet: URL:http://www.blackwell-synergy.com/doi/a bs/10.1111/j.1440-1746.2007.04895.x>

## Description

La présente demande concerne l'utilisation d'une argile pour la préparation d'un médicament destiné à traiter ou prévenir la maladie coeliaque.

La maladie coeliaque est une maladie auto-immune ; elle se caractérise par une intolérance alimentaire à certains composants du gluten tels que les prolamines. Ces dernières sont présentes en importantes quantités dans de nombreuses céréales et notamment le blé, le seigle et l'orge. La maladie coeliaque est la conséquence d'une hypersensibilité digestive à ces composants du gluten. Chez certains sujets génétiquement prédisposés, l'ingestion de cette protéine va déclencher une réaction immunitaire exagérée qui se traduit par des lésions de la muqueuse intestinale. Outre les facteurs génétiques, d'autres facteurs (infectieux, viraux et/ou bactériens) pourraient être impliqués.

La prévalence de cette maladie est très variable d'un pays à l'autre pour des raisons encore mal déterminées : par exemple, elle est de 1/100 en Europe (de 1/300 à 1/125 en Irlande) et de 1/300 aux Etats-Unis. Elle touche davantage les filles que les garçons. Les premiers signes de la maladie apparaissent très tôt dans la vie, généralement entre l'âge de six mois et de deux ans, mais ils peuvent être détectés beaucoup plus tard, parfois même à l'âge adulte.

Aujourd'hui, son traitement repose uniquement sur l'exclusion alimentaire du gluten qui, dans la majorité des cas, assure la guérison clinique et prévient les complications telles que le lymphome ou la déminéralisation osseuse. Même si le régime sans gluten semble simple dans son principe, il est très contraignant, socialement invalidant et très souvent difficile à mettre en oeuvre sans soutien médical.

La présente invention a donc pour objet l'utilisation d'une argile pour la préparation d'un médicament destiné à traiter ou prévenir la maladie coeliaque.

La présente invention a plus particulièrement pour objet l'utilisation d'une argile pour la préparation d'un médicament destiné à prévenir la maladie coeliaque.

La présente invention a plus particulièrement pour objet également l'utilisation d'une argile pour la préparation d'un médicament destiné à traiter la maladie coeliaque.

L'argile est un produit naturel connu qui trouve aujourd'hui des applications aussi bien en cosmétique que dans le domaine médical pour traiter certaines pathologies telles que les diarrhées.

Le brevet FR-A1-2 581 874, publié le 21 novembre 1986, divulgue l'utilisation de la smectite ou l'attapulgite de mormoiron (ATTA) dans des compositions à action anti-diarrhéique en combinaison avec les sels de sodium et de potassium chez des enfants âgés de 1 à 24 mois hospitalisés pour des diarrhées aiguës.

Le document de Yen, Z.-S. et al., 2006. (Emergency Medicine Journal, 23(1): 65-66) passe en revue l'efficacité de la smectite dans le cas de la diarrhée aiguë chez les enfants et conclut que la smectite raccourcit la durée de la diarrhée lorsqu'elle est combinée avec une réhydratation orale.

Yao-Zong Y et al., 2004 (Digestive and Liver Disease, 36(12) : 824-828) rapporte une étude comparative sur l'efficacité entre la smectite dioctahédrique et une préparation probiotique chez des patients présentant une diarrhée chronique fonctionnelle montrant que la smectite dioctahédrique est plus efficace que le traitement probiotique et montre une action prolongée après cessation du traitement qui peut interférer le cours de la maladie.

Gonzalez R. et al., 2004 (British Journal of Pharmacology, 141(6) : 951-960) montre l'effet anti-inflammatoire de la diosmectite (smectite dioctahédrique) sur un modèle de colite chronique induite chez le rat, et suggère un mécanisme d'action qui peut impliquer l'absorption de protéines, la protection de la muqueuse et la modulation dans la production de médiateurs pro-inflammatoires chez la muqueuse du colon.

Ducrotte P. et al, 2005 (Alimentary Pharmacology and Therapeutics, 21(4) : 435-444) rapporte l'efficacité de la montmorillonite beidellitique pour traiter le syndrome de l'intestin irritable (IBS, irritable bowel syndrome). L'étude montre que la montmorillonite beidellitique est bien tolérée chez les patients dont la constipation est le symptôme prédominant et que celle-ci induit une légère amélioration sur la douleur et le malaise.

Le brevet WO 2005/112881 A1, publié le 1 décembre 2005, divulgue des tablettes de montmorillonite pour le traitement de la diarrhée aiguë et chronique, la gastrite, l'oesophagite, la colite, l'inflammation produite par reflux gastro-intestinal, l'IBS, le déséquilibre de la flore gastro-intestinale et la douleur due aux maladies de l'estomac, de l'oesophage et du duodénum.

Theodorou V. et al., 1994 (2005 (Alimentary Pharmacology and Therapeutics, 8(3) : 295-299) rapporte l'effet protecteur de la diosmectite contre les perturbations digestives allergiques induites chez les cobayes sensibilisés avec les protéines du lait de vache.

Finalement, Leonard et al., 1994 (Gastroenterologie Clinique et Biologique, 18 (6-7) :609-616) décrit l'efficacité de la diosmectite sur les effets de la pepsine dans la muqueuse intestinale. La diosmectine est capable d'augmenter l'épaisseur de la muqueuse et de renforcer la barrière muqueuse, ainsi que d'inhiber l'effet muco-lytique de la pepsine.

L'argile utilisée selon l'invention peut être une argile de la famille des smectites. Les smectites représentent une famille particulière d'argile dans laquelle on trouve des espèces dioctaédriques telles que montmorillonite et beidellite, et des espèces trioctaédriques telle que hectorite et saponite.

L'argile utilisée selon l'invention est de préférence une smectite, et de manière très préférentielle une smectite dioctaédrique. De préférence également, la smectite dioctaédrique est une montmorillonite ou une beidellite ou une structure cristallographique intermédiaire entre les deux pôles cristallochimiques : montmorillonite et beidellite. Cette structure cristallographique intermédiaire peut être proche du pôle montmorillonite et même très proche du pôle montmorillonite ; elle peut également être proche du pôle beidellite et même très proche du pôle beidellite.

De préférence, une smectite dioctahédrique selon l'invention est une montmorillonite ou une structure intermédiaire proche du pôle montmorillonite, et de manière très préférentielle une montmorillonite ou une structure intermédiaire très proche du pôle montmorillonite.

De manière très préférentielle également, l'argile utilisée est la smectite dénommée "diosmectite" et commercialisée sous la marque Smecta^{®}.

Les compositions pharmaceutiques comprenant une argile peuvent être sous forme de solides, par exemple des poudres, des granules, des comprimés ou des gélules. Les supports solides appropriés peuvent être, par exemple, le talc, les sucres, le lactose, la dextrine, la gélatine, la cellulose et ses esters.

Les compositions pharmaceutiques comprenant une argile peuvent aussi se présenter sous forme liquide, par exemple, des solutions, des suspensions ou des sirops. Les supports liquides appropriés peuvent être, par exemple, l'eau, les solvants organiques tels que des alcools (glycérol, glycols), de même que leurs mélanges, dans des proportions variées, dans l'eau. La composition comprendra également des conservateurs tels que des esters de l'acide benzoïque.

Dans les compositions pharmaceutiques ci-dessus, l'argile peut également être associée à des colorants et/ou des arômes.

L'administration de l'argile selon l'invention peut se faire par voie orale ou entérale. De préférence, l'argile et en particulier les smectites telles que définies ci-dessus sont administrées par voie orale.

La dose d'administration journalière d'argile sera la dose habituelle recommandée pour ce produit. Dans le cas particulier de la smectite dénommée "diosmectite", elle peut être administrée à une dose journalière maximale de 18 g/jour.

A moins qu'ils ne soient définis d'une autre manière, tous les termes techniques et scientifiques utilisés ici ont la même signification que celle couramment comprise par un spécialiste ordinaire du domaine auquel appartient cette invention.

### Partie pharmacologique

Pour les patients atteints de maladie coeliaque, le diagnostic est posé par l'endoscopie oeso-gastroduodénale et les biopsies duodénales dont l'analyse histologique révèle une atrophie villositaire plus ou moins prononcée. Ainsi, l'efficacité d'une argile dans le traitement de la maladie coeliaque peut être suivie par histologie de la muqueuse intestinale.

L'activité d'une argile de type smectite telle que la smectite dénommée "diosmectite" (Smecta®) dans le traitement de la maladie coeliaque peut être évaluée selon le protocole expérimental suivant :
La première étude clinique peut être une étude de preuve d'efficacité (phase II). La smectite pourrait être administrée à la dose de 3 g trois fois par jour pendant au moins 6 mois.

Lors de l'étude, seront suivis l'état clinique, en particulier les symptômes digestifs dont la diarrhée et les ballonnements abdominaux, l'état nutritionnel en particulier biologique, les tests d'absorption des macro et micronutriments, l'état immunologique, en particulier le titre des Anticorps marqueurs de la maladie coeliaque, l'état de la muqueuse intestinale, en particulier l'amélioration de l'atrophie villositaire et de l'inflammation.

Le plan de développement comprend ensuite une étude de Phase III, randomisée en double aveugle versus placebo chez des patients dont le diagnostic est récent et chez qui le régime sans gluten est initié. L'efficacité sera appréciée par le délai de normalisation de la muqueuse intestinale, et par les mêmes paramètres que ceux suivis dans la Phase II.

## Revendications

1. Utilisation d'une argile pour la préparation d'un médicament destiné à traiter ou prévenir la maladie coeliaque, **caractérisée en ce que** l'argile est une smectite.

2. Utilisation d'une argile selon la revendication 1, pour la préparation d'un médicament destiné à prévenir la maladie coeliaque.

3. Utilisation d'une argile selon la revendication 1, pour la préparation d'un médicament destiné à traiter la maladie coeliaque.

4. Utilisation selon la revendication 1, **caractérisée en ce que** la smectite est dioctahédrique.

5. Utilisation selon la revendication 4, **caractérisée en ce que** la smectite dioctaédrique est une montmorillonite ou une beidellite ou une structure cristallographique intermédiaire entre les deux pôles cristallochimiques : montmorillonite et beidellite.

6. Utilisation selon l'une des revendications 4 ou 5, **caractérisée en ce que** la smectite dioctaédrique est une montmorillonite ou une structure cristallographique intermédiaire proche du pôle montmorillonite.

7. Utilisation selon l'une des revendications 4 à 6, **caractérisée en ce que** la smectite dioctaédrique est une montmorillonite ou une structure cristallographique intermédiaire très proche du pôle montmorillonite.

8. Utilisation selon l'une des revendications précédentes, **caractérisée en ce que** l'argile est la smectite dénommée "diosmectite".

## Patentansprüche

1. Verwendung eines Tons für die Herstellung eines Medikaments, das dazu bestimmt ist, Zöliakie zu behandeln oder zu verhindern, **dadurch gekennzeichnet, dass** der Ton ein Smektit ist.

2. Verwendung eines Tons gemäß Anspruch 1 zur Herstellung eines Medikaments, das dazu bestimmt ist, Zöliakie zu verhindern.

3. Verwendung eines Tons gemäß Anspruch 1 zur Herstellung eines Medikaments, das dazu bestimmt ist, Zöliakie zu behandeln.

4. Verwendung gemäß Anspruch 1, **dadurch gekennzeichnet, dass** der Smektit dioktaedrisch ist.

5. Verwendung gemäß Anspruch 4, **dadurch gekennzeichnet, dass** der dioktaedrische Smektit ein Montmorillonit oder ein Beidellit ist oder eine kristallografische Zwischenstruktur zwischen den zwei kristallchemischen Polen: Montmorillonit und Beidellit.

6. Verwendung gemäß einem der Ansprüche 4 oder 5, **dadurch gekennzeichnet, dass** der dioktaedrische Smektit ein Montmorillonit oder eine kristallografische Zwischenstruktur nahe dem Montmorillonit-Pol ist.

7. Verwendung gemäß einem der Ansprüche 4 bis 6, **dadurch gekennzeichnet, dass** der dioktaedrische Smektit ein Montmorillonit oder eine kristallografische Zwischenstruktur sehr nahe dem Montmorillonit-Pol ist.

8. Verwendung gemäß einem der vorangehenden Ansprüche, **dadurch gekennzeichnet, dass** der Ton der Smektit ist, der "Diosmektit" genannt wird.

## Claims

1. Use of a clay for the preparation of a medicament intended to treat or prevent coeliac disease, **characterized in that** the clay is a smectite.

2. Use of a clay according to claim 1, for the preparation of a medicament intended to prevent coeliac disease.

3. Use of a clay according to claim 1, for the preparation of a medicament intended to treat coeliac disease.

4. Use according to claim 1, **characterized in that** the smectite is dioctahedral.

5. Use according to claim 4, **characterized in that** the dioctahedral smectite is a montmorillonite or a beidellite or an intermediate crystallographic structure between the two crystal-chemical poles: montmorillonite and beidellite.

6. Use according to claim 4 or 5, **characterized in that** the dioctahedral smectite is a montmorillonite or an intermediate crystallographic structure close to the montmorillonite pole.

7. Use according to any one of claims 4 to 6, **characterized in that** the dioctahedral smectite is a montmorillonite or an intermediate crystallographic structure very close to the montmorillonite pole.

8. Use according to any one of the preceding claims, **characterized in that** the clay is the smectite called "diosmectite".
